# EUROPEAN PATENT SPECIFICATION

(11) **EP 3 080 079 B1**
(45) Date of publication and mention of the grant of the patent: **25.07.2018**
(21) Application number: 14816166.4
(22) Date of filing: 09.12.2014
(51) Int. Cl.: C07C 319/24, C07C 323/12, C10M 135/24

(54) **OXIDATION OF 2-MERCAPTOETHANOL**
OXIDATION VON 2-MERCAPTOETHANOL
OXYDATION DU 2-MERCAPTOÉTHANOL

(30) Priority: 11.12.2013 EP 13196578
(43) Date of publication of application: 19.10.2016
(73) Proprietor: BASF SE, 67056 Ludwigshafen am Rhein (DE)
(72) Inventor: VAUTRAVERS, Nicolas, 68159 Mannheim (DE); TELES, Joaquim Henrique, 67165 Waldsee (DE)
(74) Representative: BASF IP Association
(86) International application number: PCT/EP2014/076983
(87) International publication number: WO 2015/086565

(56) References cited:
- US-A- 4 606 833
- US-A1- 2006 142 616
- HADI ADIBI ET AL: "Iron(III) Trifluoroacetate: Chemoselective and Recyclable Lewis Acid Catalyst for Diacetylation of Aldehydes, Thioacetalization and Transthioacetalization of Carbonyl Compounds and Aerobic Coupling of Thiols", CHINESE JOURNAL OF CHEMISTRY, vol. 26, no. 11, 1 November 2008 (2008-11-01), pages 2086-2092, XP055175575, ISSN: 1001-604X, DOI: 10.1002/cjoc.200890372 cited in the application
- ANUPAMA PARMAR ET AL: "Iron (III) Perchlorate Adsorbed on Silica Gel: A Reagent for Organic Functional Group Transformations", SYNTHETIC COMMUNICATIONS, vol. 37, no. 14, 1 July 2007 (2007-07-01), pages 2301-2308, XP055175576, ISSN: 0039-7911, DOI: 10.1080/00397910701410772

## Description

The present invention relates to a process for the preparation of bis-(2-hydroxyethyl)-disulfide by oxidizing 2-mercaptoethanol with oxygen in a reaction mixture comprising at least one homogeneously distributed iron comprising salt or complex as catalyst and at least one tertiary amine.

Processes for the preparation of bis-(2-hydroxyethyl)-disulfide have already been mentioned in the prior art.

US 4,258,212 and US 5,659,086 describe the oxidation of 2-mercaptoethanol to bis-(2-hydroxyethyl)-disulfide with hydrogen peroxide in the presence of a base. According to US 4,258,212, an alkali metal hydroxide like sodium hydroxide is required to control the pH within a range from 7 to 9. US 5,659,086 teaches the use of an inorganic or organic base, including alkali metal hydroxides like sodium hydroxide as well as primary, secondary or tertiary amines. A decisive disadvantage of these processes is the use of hydrogen peroxide, due to its high price. Because of its high oxidation capability, hydrogen peroxide also requires careful handling during transport, storage and in particular during its application as oxidizing agent. A further disadvantage of hydrogen peroxide is its high dilution with water. Technical hydrogen peroxide usually has only a content of 50% by weight of H₂O₂ for safety reasons. The rest is water, beside traces of a stabilizer, which decreases the efficiency of the oxidation reaction and which leads to a highly diluted reaction product. As consequence the reaction product has to be further processed to obtain the bis-(2-hydroxyethyl)-disulfide with a low water content.

US 2004/0116748 A1 discloses the oxidation of mercaptans like 2-mercaptoethanol to the corresponding disulfides by using elemental sulfur as oxidizing agent. A decisive disadvantage of this process is the stoichiometric formation of hydrogen sulfide, which is highly toxic and which has to be removed carefully from the reaction product and disposed.

Y. Wang et al. in Biomacromolecules 2011, 12, 66 to 74, mention the synthesis of bis-(2-hydroxyethyl) disulfide by oxidation of 2-mercaptoethanol using dimethyl sulfoxide as oxidizing agent. A decisive disadvantage of this process is the requirement of dimethyl sulfoxide as oxidation agent, which has to be produced in a complex process. Another decisive disadvantage is the stoichiometric formation of dimethyl sulfide which is a stench and has to be removed from the reaction product and disposed.

S. Murata et al. in Journal of Chemical Society Perkin Transactions, 1989, 617 to 621, disclose the conversion of aromatic nitro compounds with 2-mercaptoethanol in the presence of a iron complex or iron salt and a solvent into the corresponding aromatic amine and bis-(2-hydroxyethyl}-disulfide. A decisive disadvantage of this process is the use of aromatic nitro compounds as the oxidation agent. Aromatic nitro compounds are only accessible through complex processes and are usually toxic or even explosive. Another decisive disadvantage is the stoichiometric formation of the corresponding aromatic amines, which are usually also toxic and which have to be removed from the reaction product and disposed.

Another oxidation agent for oxidizing 2-mercapto ethanol into bis-(2-hydroxyethyl)-disulfide described in the state of the art is oxygen, like pure oxygen or air. The use of oxygen has the general advantage that only small amounts of water are produced as oxidation byproduct.

US 2006/0142616 A1 and its German equivalent DE 103 23 839 B3 teach the preparation of bis-(2-hydroxyethyl)-disulfide by oxidation of 2-mercaptoethanol with oxygen in the presence of a copper salt or a manganese salt as catalyst and ammonia or a primary, secondary or tertiary amine as co-catalyst. The conversion is claimed to be nearly 100% without leading to byproducts. According to the teaching of this document, no extensive purification steps are required, but the added ammonia and amines, respectively, are distilled off together with water. Even if the used oxidation agent, the reaction conditions, the conversion and the yield of bis-(2-hydroxyethyl)-disulfide according to this document seem to be beneficial, the applied catalysts show some disadvantages. Copper and manganese are rare, not easy to mine and therefore relatively expensive. Furthermore, copper is well known to be toxic, in particular if absorbed through solid food or fluids like drinking water.

H. Adibi et al. in Chinese Journal of Chemistry, 2008, 26, 2086 to 2092, teach the conversion of 2-mercaptoethanol into bis-(2-hydroxyethyl)-disulfide by oxidation with oxygen in the presence of iron(III) trifiuoroacetate and sodium iodide. The conversion is described to be very high with a yield of 97%. The thiol/catalyst/Nal ratio used in the experiment was 1/0.1/0.2, corresponding to a high concentration of iron(III)trifluoroacetate catalyst of 10 mol-% and a high concentration of sodium iodide of 20 mol-%, whereby both percentage values are in relation to 2-mercaptoethanol.

The document "A. Parmar et al., Synthetic Communications, vol. 37, no. 14, pages 2301 to 2308" discloses a process for the oxidation of alkyl/aryl thiols to disulfides using iron(III)perchlorate adsorbed on silica gel.

US 4,606,833 A discloses the use of bis-(2-hydroxyethyl)-disulfide as lubricating additive, but processes for its preparation are not disclosed in this document.

The object of the present invention is therefore to provide a process for the preparation of bis-(2-hydroxyethyl)-disulfide starting with 2-mercaptoethanol using an oxidant which leaves the smallest possible amount of water or other substances in the crude product. Further, a process shall be provided giving rise to the desired product in high yield and high purity, preferably without additional purification steps. A catalyst shall be used in the process containing at least one metal that is not toxic for animals, human-beings and/or the environment and has therefore not to be separated from the reaction mixture after completion of the reaction.

These objects are solved by the process for the preparation of bis-(2-hydroxyethyl)-disulfide by oxidizing 2-mercaptoethanol with oxygen in a reaction mixture comprising at least one homogeneously distributed iron comprising salt or complex as catalyst and at least one tertiary amine, according to the present invention.

The process according to the present invention is conducted to obtain bis-(2-hydroxyethyl)-disulfide by oxidizing 2-mercaptoethanol. The reaction, which is in general known to the skilled artisan, is shown in the following:

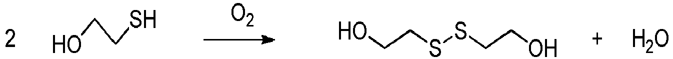

The substrate of the process according to the present invention is 2-mercaptoethanol. It can be prepared using processes that are known to the skilled artisan, for example by the addition of H₂S to ethylene oxide. Further, 2-mercaptoethanol is also commercially available.

In general, 2-mercaptoethanol can be used with a purity that is typical for chemical compounds that are used in chemical reactions. Preferably, 2-mercaptoethanol is used in the process according to the present invention with a purity of at least 95 % by weight, more preferably at least 98 % by weight.

The desired product that is obtained with the process according to the present invention, is bis-(2-hydroxyethyl)-disulfide and is also in general known to the skilled in the art. According to the present invention, crude bis-(2-hydroxyethyl)-disulfide is in general obtained with a purity of at least 80 % by weight, more preferably at least 85 % by weight.

In the process according to the present invention, oxygen is used as the oxidant. According to the present invention oxygen can be added in pure form as a gas. In addition, it is also possible that oxygen is used as a mixture with further gases, preferably with gases that are inert towards the chemical compounds that are present in the reaction mixture according to the present invention.

Suitable gases that may be present in mixtures comprising oxygen that are used according to the present invention are preferably selected from the group consisting of carbon dioxide, noble gases like helium, argon, nitrogen and mixtures thereof.

According to a preferred embodiment of the process according to the present invention, air is used as an oxygen comprising gas. In general, air comprises nitrogen, oxygen, argon, and further gases in minor amounts.

The amount of oxygen that is used in the process according to the present invention is in general adjusted by the pressure of oxygen, in particular by the partial pressure of oxygen in the gas that is used.

In general, the process according to the present invention can be conducted at any partial pressure of oxygen that is suitable, in particular in respect of reaction rate, amount of side products etc.

The process according to the present invention is preferably conducted at a partial pressure of oxygen of 0.2 to 20 bar (a), particularly preferably 1 to 10 bar (a)

The present invention therefore preferably relates to the process according to the present invention, wherein it is conducted at a partial pressure of oxygen of 0.2 to 20 bar (a), particularly preferably 1 to 10 bar (a)

According to the preferred embodiment of the process according to the present invention, wherein air is used as an oxygen comprising gas, the process of the present invention is conducted at a pressure of 1 to 30 bar (a), preferably 5 to 25 bar (a), more preferably 10 to 20 bar (a).

The reaction mixture that is used in the process according to the present invention comprises 2-mercaptoethanol, bis-(2-hydroxyethyl)-disulfide, at least one tertiary amine, at least one iron comprising salt or complex and oxygen.

The present invention therefore preferably relates to the process according to the present invention, wherein the reaction mixtures comprises 2-mercaptoethanol, bis-(2-hydroxyethyl)-disulfide, at least one tertiary amine, at least one iron comprising salt or complex and oxygen.

According to a preferred embodiment of the process according to the present invention, the reaction mixture does not comprise any further components beside substrate, product, catalyst, tertiary amine and oxygen. During the reaction, water is prepared from the oxidant, yielding a reaction mixture that further comprises water in minor amounts. In addition, if air is used as oxygen containing gas, gases like nitrogen and argon are also present in the mixture.

The present invention therefore preferably relates to the process according to the present invention, wherein the reaction mixture consists of 2-mercaptoethanol, bis-(2-hydroxyethyl)-disulfide, at least one tertiary amine, at least one iron comprising salt or complex, water, oxygen and optionally further components like nitrogen and argon, more preferably the reaction mixture consists of 2-mercaptoethanol, bis-(2-hydroxyethyl)-disulfide, at least one tertiary amine, at least one iron comprising salt or complex, water and oxygen.

The present invention therefore preferably relates to the process according to the present invention, wherein the reaction mixture consists of 2-mercaptoethanol, bis-(2-hydroxyethyl)-disulfide, at least one tertiary amine, at least one iron comprising salt or complex, water, oxygen and optionally further components.

Further preferred, the process according to the present invention is conducted in absence of any solvent, preferably in absence of water and/or any organic solvent. According to the present invention, "in absence of any solvent, preferably in absence of water and/or any organic solvent" means that the amount of solvent like water and/or organic solvent is less than 10 % by weight, preferably less than 5 % by weight.

The process according to the present invention is conducted in the presence of at least one homogeneously distributed iron comprising salt or complex as catalyst.

In general, all iron comprising salts or complexes that are able to be homogeneously distributed in the reaction mixture, may be used. Due to the fact, that the reaction mixture predominantly comprises organic compounds, in particular 2-mercaptoethanol, bis-(2-hydroxyethyl)-disulfide and at least one tertiary amine, the iron salt or complex should be homogeneously distributable in this medium.

Therefore, preferably at least one iron salt or complex is used in the process according to the present invention, having a solubility of at least 0.1 mmol/l, particularly preferably at least 0.2 mmol/l, more preferably at least 0.5 mmol/l, in each case in the above mentioned media and in each case in respect of the whole reaction mixture. An upper limit of the solubility of the at least one iron salt or complex that is used in the process according to the present invention is for example 1.0 mol/l.

Further preferred, the at least one iron salt or complex is selected from organic or inorganic iron salts or complexes.

The present invention therefore preferably relates to the process according to the present invention, wherein the at least one iron salt or complex is selected from organic or inorganic iron salts or complexes.

Further preferred, the iron being present in the at least one iron salt or complex that is used as catalyst may have any suitable oxidation state like 0, +2 and/or +3, preferably +2 and/or +3.

The present invention therefore preferably relates to the process according to the present invention, wherein the at least one iron salt comprises iron in the oxidation state +2 and/or +3.

Particularly suitable iron salts or complexes, optionally containing at least one neutral ligand, like water, are selected from the group consisting of iron(II) oxide, iron(III) oxide, iron (II,III) oxide, iron(II) sulphide, iron(II) disulphide, iron(II,III) sulphide, lithium iron(II) phosphate, lithium iron(III) oxide, iron(II) phosphide, iron(III) phosphide, iron(III) pyrophosphate, iron(III) phosphate, iron(III) ionophore IV, iron(II) molybdate, ammonium iron(III) hexacyanoferrate(II), iron(III) ferrocyanide, 5,10,15,20-Tetrakis(pentafluorophenyl)-21H, 23H-porphyrin iron(III) chloride, 5,10,15,20-Tetraphenyl-21H, 23H-porphine iron(III) chloride, 5,10,15,20-tetrakis(4-methoxyphenyl)-21H, 23H-porphine iron(III) chloride, 2, 3 , 7, 8, 12, 13 , 17, 18-octaethyl-21h,23h-porphine iron(III) acetate, 2,3,7,8,12,13,17,18-Octaethyl-21H,23H-porphine iron(III) chloride, acetyl-cyclopentadienyl-iron(II) carbonyl triphenylphosphine complex, [N,N'-Bis(2-pyridylmethyl)]-2,2'-bipyrrolidinebis(acetonitrile)iron(II) hexafluoroantimonate, iron(II) titanate, iron(II) silicide, iron(III) ionophore VI, bis(cyclopentadienyl) iron(II), cyclopentadienyl(formylcyclopentadienyl)iron(II), (boronocyclopentadienyl)cyclopentadienyl iron(II), bis(cyclopentadienyl)iron(III) tetrafluoroborate, cyclopentadienyl[(hydroxymethyl) cyclopentadienyl] iron(II), bis(acetylcyclopentadienyl)iron(II), bis(methylcyclopentadienyl)iron(II), bis(ethylcyclopentadienyl)iron(II), bis(i-propylcyclopentadienyl)iron(II), dichloro-bis[o-phenylenebis(diphenylphosphine)]iron(II), cyclopentadienyliron(II) dicarbonyl iodide, bis(pentamethylcyclopentadienyl)iron(II), bis(tetramethylcyclopentadienyl)iron(II), cyclopentadi-enyldicarbonyl(tetrahydrofuran)iron(II), tetrafluoroborate tricarbonyl(cyclooctatetraene)iron(II), cyclopentadienyl iron(II) dicarbonyl dimer, cyclopentadienyl(fluorene)iron(II) hexafluorophosphate, (nicotinamidomethyl)phosphonic acid iron(II) salt, tricarbonyl(2-methoxycyclohexadienylium) iron(II) hexafluorophosphate, tricarbonyl(4-methoxy-1-methylcyclohexadienylium)iron(II) tetrafluoroborate, meso-tetra(4-N-methypyridyl)porphyrine iron(III), 4-dimethylaminopyridinyl(pentaphenylcyclopentadienyl) iron (II), 4-pyrrolidinopyrindinyl(pentamethylcyclopentadienyl) iron(II), 2,6-Bis-[1-(2,6-diisopropylphenylimino)ethyl]pyridine iron (II) chloride, (aminomethyl)-phosphonic acid, iron(II) salt, bis[(1E)-N-(aminocarbothioyl)ethanehydrazonoyl]iron(II), bis[(1E)-N-(anilinocarbothioyl)ethanehydrazonoyl]iron(II), bis[(E)-(aminocarbothioyl)hydrazono](phenyl)methyl]iron(II), iron(III) i-propoxide, iron(II) acetate, iron(III) oxo acetate perchlorate, ammonium iron(III) citrate, iron(II) acetylacetonate, iron(III) acetylacetonate, iron(II) bromide, iron(III) bromide, iron(II) chloride, iron(III) chloride, iron(II) arsenide, iron(III) arsenide, iron(III) citrate, iron(II) phthalocyanine bis(pyridine) complex, iron(II) ethylendiammoniumsulfate, iron(II) oxalate, iron(III) oxalate, ammonium iron(III) oxalate, iron(II) fluoride, iron(III) fluoride, iron(II) fumarate, iron(II) gluconate, iron(II) iodide, iron(III) iodide, iron(II) lactate, iron(III) nitrate, iron(II) phthalocyanine, iron(III) phthalocyanine-4,4',4",4"'-tetrasulfonic acid, iron(III) phthalocyanine chloride, iron(II) perchlorate, iron(III) perchlorate, iron(II) sulphate, iron(III) sulphate, ammonium iron(II) sulphate, ammonium iron(III) sulphate, iron(II) phosphate, iron(III) phosphate, iron(III) tartrate, (+)-iron(II) ascorbate, iron(II) stearate, iron(II) sulfamate, iron(II) tetrafluoroborate, tetraethylammonium tetrachloroiron(III), tris(dibenzoylmethanato)iron(III), tris(ethylenediamine)iron(II) sulfate, iron(III) p-toluenesulfonate, iron(II) trifluoromethanesulfonate, iron(III) trifluoromethanesulfonate, iron(III) trifluoroacetylacetonate, bis(o-phenanthroline)iron(II) cyanide, bis(hexafluoroacetylacetonato)-(N,N,N',N'-tetramethylethylenediamine)iron (II), ethylenediaminetetraacetic acid, iron(III) sodium salt, diethylenetriaminepentaacetic acid iron(III) disodium salt, bis(N,N'-di-t-butyl acetamidina-to)iron(II), iron 2-ethylhexanoate, iron(III) naphthenate, tris(2,2,6,6-tetramethyl-3,5-heptanedionato)iron(III) and mixtures thereof.

The present invention therefore preferably relates to the process according to the present invention, wherein the at least one iron salt or complex, optionally containing at least one neutral ligand, like water, is selected from the group consisting of iron(II) oxide, iron(III) oxide, iron (II,III) oxide, iron(II) sulphide, iron(II) disulphide, iron(II,III) sulphide, lithium iron(II) phosphate, lithium iron(III) oxide, iron(II) phosphide, iron(III) phosphide, iron(III) pyrophosphate, iron(III) phosphate, iron(III) ionophore IV, iron(II) molybdate, ammonium iron(III) hexacyanoferrate(II), iron(III) ferrocyanide, 5,10,15,20-Tetrakis(pentafluorophenyl)-21H, 23H-porphyrin iron(III) chloride, 5,10,15,20-Tetraphenyl-21H, 23H-porphine iron(III) chloride, 5,10,15,20-tetrakis(4-methoxyphenyl)-21H, 23H-porphine iron(III) chloride, 2,3,7,8,12,13,17,18-octaethyl-21h,23h-porphineiron(III)acetate, 2,3,7,8,12,13,17,18-Octaethyl-21H,23H-porphine iron(III) chloride, acetyl-cyclopentadienyl-iron(II) carbonyl triphenylphosphine complex, [N,N'-Bis(2-pyridylmethyl)]-2,2'-bipyrrolidinebis(acetonitrile)iron(II) hexafluoroantimonate, iron(II) titanate, iron(II) silicide, iron(III) ionophore VI, bis(cyclopentadienyl) iron(II), cyclopentadienyl(formylcyclopentadienyl)iron(II), (boronocyclopentadienyl)cyclopentadienyl iron(II), bis(cyclopentadienyl)iron(III) tetrafluoroborate, cyclopentadienyl[(hydroxymethyl) cyclopentadienyl] iron(II), bis(acetylcyclopentadienyl)iron(II), bis(methylcyclopentadienyl)iron(II), bis(ethylcyclopentadienyl)iron(II), bis(i-propylcyclopentadienyl)iron(II), dichloro-bis[o-phenylenebis(diphenylphosphine)]iron(II), cyclopentadienyliron(II) dicarbonyl iodide, bis(pentamethylcyclopentadienyl)iron(II), bis(tetramethylcyclopentadienyl)iron(II), cyclopentadi-enyldicarbonyl(tetrahydrofuran)iron(II), tetrafluoroborate tricarbonyl(cyclooctatetraene)iron(II), cyclopentadienyl iron(II) dicarbonyl dimer, cyclopentadienyl(fluorene)iron(II) hexafluorophosphate, (nicotinamidomethyl)phosphonic acid iron(II) salt, tricarbonyl(2-methoxycyclohexadienylium) iron(II) hexafluorophosphate, tricarbonyl(4-methoxy-1-methylcyclohexadienylium)iron(II) tetrafluoroborate, meso-tetra(4-N-methypyridyl)porphyrine iron(III), 4-dimethylaminopyridinyl(pentaphenylcyclopentadienyl) iron (II), 4-pyrrolidinopyrindinyl(pentamethylcyclopentadienyl) iron(II), 2,6-Bis-[1-(2,6-diisopropylphenylimino)ethyl]pyridine iron (II) chloride, (aminomethyl)-phosphonic acid, iron(II) salt, bis[(1E)-N-(aminocarbothioyl)ethanehydrazonoyl]iron(II), bis[(1E)-N-(anilinocarbothioyl)ethanehydrazonoyl]iron(II), bis[(E)-(aminocarbothioyl)hydrazono](phenyl)methyl]iron(II), iron(III) i-propoxide, iron(II) acetate, iron(III) oxo acetate perchlorate, ammonium iron(III) citrate, iron(II) acetylacetonate, iron(III) acetylacetonate, iron(II) bromide, iron(III) bromide, iron(II) chloride, iron(III) chloride, iron(II) arsenide, iron(III) arsenide, iron(III) citrate, iron(II) phthalocyanine bis(pyridine) complex, iron(II) ethylendiammoniumsulfate, iron(II) oxalate, iron(III) oxalate, ammonium iron(III) oxalate, iron(II) fluoride, iron(III) fluoride, iron(II) fumarate, iron(II) gluconate, iron(II) iodide, iron(III) iodide, iron(II) lactate, iron(III) nitrate, iron(II) phthalocyanine, iron(III) phthalocyanine-4, 4', 4", 4"'-tetrasulfonic acid, iron(III) phthalocyanine chloride, iron(II) perchlorate, iron(III) perchlorate, iron(II) sulphate, iron(III) sulphate, ammonium iron(II) sulphate, ammonium iron(III) sulphate, iron(II) phosphate, iron(III) phosphate, iron(III) tartrate, (+)-iron(II) ascorbate, iron(II) stearate, iron(II) sulfamate, iron(II) tetrafluoroborate, tetraethylammonium tetrachloroiron(III), tris(dibenzoylmethanato)iron(III), tris(ethylenediamine)iron(II) sulfate, iron(III) p-toluenesulfonate, iron(II) trifluoromethanesulfonate, iron(III) trifluoromethanesulfonate, iron(III) trifluoroacetylacetonate, bis(o-phenanthroline)iron(II) cyanide, bis(hexafluoroacetylacetonato)-(N,N,N',N'-tetramethylethylenediamine)iron (II), ethylenediaminetetraacetic acid, iron(III) sodium salt, diethylenetriaminepentaacetic acid iron(III) disodium salt, bis(N,N'-di-t-butyl acetamidina-to)iron(II), iron 2-ethylhexanoate, iron(III) naphthenate, tris(2,2,6,6-tetramethyl-3,5-heptanedionato)iron(III) and mixtures thereof.

Particularly preferably, the at least one iron salt or complex is selected from the group consisting of Fe(III)-salts, in particular it is selected from the group consisting of Fe(NO₃)₃ + 9 H₂O, Fe₂(SO₄)₃, Fe(acetylacetonate)₃, FeCl₃ + 6 H₂O and mixtures thereof.

In general the at least one iron salt or complex may be used in any amount which is suitable. Preferably, the catalyst is present in an amount of 0.1 to 50 µmol iron, preferably 1 to 40 µmol iron, particularly preferably 5 to 20 µmol iron, in each case per mol 2-mercaptoethanol.

The present invention therefore preferably relates to the process according to the present invention, wherein the catalyst is present in an amount of 0.1 to 50 µmol iron, preferably 1 to 40 µmol iron, particularly preferably 5 to 20 µmol iron, in each case per mol 2-mercaptoethanol.

The reaction according to the present invention is further conducted in the presence of at least one tertiary amine.

In general, any tertiary amine that is known to the skilled artisan can be used in the process according to the present invention. According to the present invention, the at least one tertiary amine acts as a basic cocatalyst.

According to a preferred embodiment of the present invention, the at least one tertiary amine contains three identical or different, unbranched or branched alkyl radicals having 1 to 20 carbon atoms in each case, where individual carbon atoms can also be, independently of another, replaced by a hetero atom selected from the group consisting of N or O and two or three radicals can also be joined to one another to form a chain comprising at least four atoms.

The present invention therefore preferably relates to the process according to the present invention, wherein the at least one tertiary amine contains three identical or different, unbranched or branched alkyl radicals having 1 to 20 carbon atoms in each case, where individual carbon atoms can also be, independently of another, replaced by a hetero atom selected from the group consisting of N or O and two or three radicals can also be joined to one another to form a chain comprising at least four atoms.

According to a particularly preferred embodiment of the process according to the present invention, the at least one tertiary amine is selected from the group consisting of trimethyl amine, triethyl amine, tripropyl amine, triisopropyl amine, ethyl diisopropyl amine, tri-n-butyl amine, tripentyl amine, trihexyl amine, tricyclohexyl amine, triisoamyl amine, trioctyl amine, tris(2-ethylhexyl) amine, tristearyl amine, trioleyl amine, tridecyl amine, dimethyl stearyl amine, N,N-dimethyl benzyl amine, N,N-dibutyl benzyl amine, N,N-dimethyl aniline, N,N-dihexyl aniline, N,N-diethyl aniline, N,N-dimethyltoluidine, pyridine, quinoline, picoline, 2,4-lutidine, 2,6-lutidine, trimethylpyridine, 2-methyl-5-ethylpyridine (collidine), N-methylpiperidine, N,N'-dimethylpiperazine, N-methyl morpholine, N-methyl pyrrolidine, sparteine, tris(2-hydroxyethyl) amine, tris(2-hydroxypropyl) amine, methyl di(2-hydroxyethyl) amine, (N,N-Dimethylaminopropyl)-acetamide, octyldiethyl amine, N-octyl-N-hydroxyethylmethylamine, N,N-didecylmethyl amine, N-dodecyl-N-tetradecylhydroxyethylamine, N,N-ditetradecylmethylamine, N-tetradecyldimethylamine, N-hexadecyl-N-ethylmethylamine, N-octadecyl-N-eicosylmethylamine, N-docosyldimethylamine, N-tetracosyldimethylamine, triethylenediamine, tetramethyl guanidine, DABCO, N,N,N',N",N"-Pentamethyldiethylenetriamine, N,N,N',N'-Tetraethyl-1,3-propanediamine, N,N,N',N'-Tetramethyl-1,4-butanediamine, N,N,N',N'-Tetramethyl-2-butene-1,4-diamine, N,N,N',N'-Tetramethyl-1,6-hexanediamine, 1,4,8,11-Tetramethyl-1,4,8,11-tetraazacyclotetradecane, 1,3,5-Trimethylhexahydro-1,3,5-triazine, DBU, DBN and mixtures thereof.

The present invention therefore preferably relates to the process according to the present invention, wherein the at least one tertiary amine is selected from the group consisting of trimethyl amine, triethyl amine, tripropyl amine, triisopropyl amine, ethyl diisopropyl amine, tri-n-butyl amine, tripentyl amine, trihexyl amine, tricyclohexyl amine, triisoamyl amine, trioctyl amine, tris(2-ethylhexyl) amine, tristearyl amine, trioleyl amine, tridecyl amine, dimethyl stearyl amine, N,N-dimethyl benzyl amine, N,N-dibutyl benzyl amine, N,N-dimethyl aniline, N,N-dihexyl aniline, N,N-diethyl aniline, N,N-dimethyltoluidine, pyridine, quinoline, picoline, 2,4-lutidine, 2,6-lutidine, trimethylpyridine, 2-methyl-5-ethylpyridine (collidine), N-methylpiperidine, N,N'-dimethylpiperazine, N-methyl morpholine, N-methyl pyrrolidine, sparteine, tris(2-hydroxyethyl) amine, tris(2-hydroxypropyl) amine, methyl di(2-hydroxyethyl) amine, (N,N-Dimethylaminopropyl)-acetamide, octyldiethyl amine, N-octyl-N-hydroxyethylmethylamine, N,N-didecylmethyl amine, N-dodecyl-N-tetradecylhydroxyethylamine, N,N-ditetradecylmethylamine, N-tetradecyldimethylamine, N-hexadecyl-N-ethylmethylamine, N-octadecyl-N-eicosylmethylamine, N-docosyldimethylamine, N-tetracosyldimethylamine, triethylenediamine, tetramethyl guanidine, DABCO, N,N,N',N",N"-Pentamethyldiethylenetriamine, N,N,N',N'-Tetraethyl-1,3-propanediamine, N,N,N',N'-Tetramethyl-1,4-butanediamine, N,N,N',N'-Tetramethyl-2-butene-1,4-diamine, N,N,N',N'-Tetramethyl-1,6-hexanediamine, 1,4,8,11-Tetramethyl-1,4,8,11-tetraazacyclotetradecane, 1,3,5-Trimethylhexahydro-1,3,5-triazine, DBU, DBN and mixtures thereof.

Most preferably, the at least one tertiary amine is tri-n-butyl-amine.

In general, the at least one tertiary amine may be added in any amount to the reaction mixture according to the present invention, as long as it provides advantages to the process.

According to a preferred embodiment of the process according to the present invention the at least one tertiary amine is added in an amount of 0.01 to 10 mol%, preferably 0.05 to 5 mol%, particularly preferably 0.07 to 3 mol%, in each case based on the amount of 2-mercaptoethanol.

In general, the process according to the present invention can be conducted at any suitable temperature, preferably the process according to the present invention is conducted at a temperature of 0 to 100 °C, more preferably 10 to 80 °C, particularly preferably 20 to 60 °C.

The present invention therefore preferably relates to the process according to the present invention, wherein it is conducted at a temperature of 0 to 100 °C more preferably 10 to 80 °C, particularly preferably 20 to 60 °C.

The process according to the present invention can be conducted continuously or batchwise.

The process according to the present invention can be conducted in any apparatus known to the skilled artisan and suitable for performing a reaction between a liquid and a gas, like stirred tank reactor, a bubble column or a jet-loop reactor.

The desired product that is obtained from the process according to the present invention can be worked-up, in particular purified, according to any method known to the skilled art, like extraction, distillation etc. According to a preferred embodiment of the process according to the present invention, the product obtained from the process has not to be worked-up, in particular purified, but can be used directly after being prepared.

The process according to the present invention gives rise to bis-(2-hydroxyethyl)-disulfide having very specific features, compared to bis-(2-hydroxyethyl)-disulfide obtainable by other processes like low water content and is free of toxic metals. For example, the bis-(2-hydroxyethyl)-disulfide that is obtainable by the process according to the present invention has a preferably low amount of water of less than 15 % by weight.

Based on these advantageous properties, bis-(2-hydroxyethyl)-disulfide obtainable, preferably obtained, with the process according to the present invention can be used as intermediate in the manufacture of chemical compounds, such as lubricant additives and in the tertiary oil recovery.

The term "Tertiary Oil recovery" is in general known to the skilled artisan and is a generic term for techniques for increasing the amount of crude oil that can be extracted from an oil field.

Tertiary oil recovery can be accomplished by the injection of various chemicals, usually as dilute solutions. These chemicals are used to aid mobility and the reduction in surface tension. According to the present invention bis-(2-hydroxyethyl)-disulfide is injected to lower the interfacial tension or capillary pressure that impedes oil droplets from moving through a reservoir. According to preferred embodiment, bis-(2-hydroxyethyl)-disulfide is injected into several wells and the production occurs in other nearby wells.

### Examples

### Examples 1 to 7 (according to the present invention)

A 270 mL autoclave is filled with 2-mercaptoethanol (1.43 mol), tri-n-butylamine (0.15 mol%) and the respective iron salt (6.5*10⁻⁶ mol Fe per mol 2-mercaptoethanol) and heated at 40°C under a constant air atmosphere (15 bar). After 24 hours, the reaction mixture is analyzed by ¹H-NMR.

### Example 8 (comparative):

The reaction is conducted according to examples 1 to 7 according to the present invention, but without tri-n-butyl amine.

The results of examples 1 to 7 and 8 are shown in the following table:

**Table 1:**

| **Example** | **Fe(III) salt** | **Yield (%)** |
|---|---|---|
| **1** | Fe(NO₃)₃ + 9 H₂O | 91 |
| **2** | Fe₂(SO₄)₃ | 93 |
| **3** | Fe(acetylacetonate)₃ | 95 |
| **4** | FeCl₃ + 6 H₂O | 95 |
| **5** | FeCl₃ + 6 H₂O ¹⁾ | 98 |
| **6** | FeCl₃ + 6 H₂O ²⁾ | 99 |
| **7** | FeCl₃ + 6 H₂O ³⁾ | 98 |
| **8 (comparative)** | FeCl₃ + 6 H₂O | <10% |

| | | |
|---|---|---|
| 1) 1.3*10⁻⁵ mol Fe per mol 2-mercaptoethanol and 0.15 mol% of n-tributylamine 2) 30 bar 3) 30 bar, 16 hour | | |

### Example 9 (comparative, according to DE 10323839 B3):

A 270 mL autoclave is filled with 2-mercaptoethanol (1.43 mol), tri-n-butylamine (0.15 mol %) and MnSO₄ (6.5×10⁻⁶ mol Mn per mol 2-mercaptoethanol) and heated at 40°C under a constant air atmosphere (15 bar). After 24 hours, 97% yield is observed by ¹H-NMR.

## Claims

1. A process for the preparation of bis-(2-hydroxyethyl)-disulfide by oxidizing 2-mercaptoethanol with oxygen in a reaction mixture comprising at least one homogeneously distributed iron comprising salt or complex as catalyst and at least one tertiary amine.

2. The process according to claim 1, wherein the at least one iron salt or complex is selected from organic or inorganic iron salts or complexes.

3. The process according to claim 1 or 2, wherein the at least one iron salt comprises iron in the oxidation state +2 and/or +3.

4. The process according to any of claims 1 to 3, wherein the at least one iron salt or complex, optionally containing at least one neutral ligand, like water, is selected from the group consisting of iron(II) oxide, iron(III) oxide, iron (II,III) oxide, iron(II) sulphide, iron(II) disulphide, iron(II,III) sulphide, lithium iron(II) phosphate, lithium iron(III) oxide, iron(II) phosphide, iron(III) phosphide, iron(III) pyrophosphate, iron(III) phosphate, iron(III) ionophore IV, iron(II) molybdate, ammonium iron(III) hexacyanoferrate(II), iron(III) ferrocyanide, 5,10,15,20-Tetrakis(pentafluorophenyl)-21H, 23H-porphyrin iron(III) chloride, 5,10,15,20-Tetraphenyl-21H, 23H-porphine iron(III) chloride, 5,10,15,20-tetrakis(4-methoxyphenyl)-21H, 23H-porphine iron(III) chloride, 2, 3 , 7, 8, 12, 13 , 17, 18-octaethyl-21h,23h-porphine iron(III) acetate, 2,3,7,8,12,13,17,18-Octaethyl-21H,23H-porphine iron(III) chloride, acetyl-cyclopentadienyl-iron(II) carbonyl triphenylphosphine complex, [N,N'-Bis(2-pyridylmethyl)]-2,2'-bipyrrolidinebis(acetonitrile)iron(II) hexafluoroantimonate, iron(II) titanate, iron(II) silicide, iron(III) ionophore VI, bis(cyclopentadienyl) iron(II), cyclo-pentadienyl(formylcyclopentadienyl)iron(II), (boronocyclopentadienyl)cyclopentadienyl iron(II), bis(cyclopentadienyl)iron(III) tetrafluoroborate, cyclopentadienyl[(hydroxymethyl) cyclopentadienyl] iron(II), bis(acetylcyclopentadienyl)iron(II), bis(methylcyclopentadienyl) iron(II), bis(ethylcyclopentadienyl)iron(II), bis(i-propylcyclopentadienyl)iron(II), dichloro-bis[o-phenylenebis(diphenylphosphine)]iron(II), cyclopentadienyliron(II) dicarbonyl iodide, bis(pentamethylcyclopentadienyl)iron(II), bis(tetramethylcyclopentadienyl)iron(II), cyclo-pentadienyldicarbonyl(tetrahydrofuran)iron(II), tetrafluoroborate tricarbonyl(cyclooctatetraene)iron(II), cyclopentadienyl iron(II) dicarbonyl dimer, cyclopentadienyl(fluorene)iron(II) hexafluorophosphate, (nicotinamidomethyl)phosphonic acid iron(II) salt, tricarbonyl(2-methoxycyclohexadienylium) iron(II) hexafluorophosphate, tricarbonyl(4-methoxy-1-methylcyclohexadienylium)iron(II) tetrafluoroborate, meso-tetra(4-N-methypyridyl)porphyrine iron(III), 4-dimethylaminopyridinyl(pentaphenylcyclopentadienyl) iron (II), 4-pyrrolidinopyrindinyl(pentamethylcyclopentadienyl) iron(II), 2,6-Bis-[1-(2,6-diisopropylphenylimino)ethyl]pyridine iron (II) chloride, (aminomethyl)-phosphonic acid, iron(II) salt, bis[(1E)-N-(aminocarbothioyl)ethanehydrazonoyl]iron(II), bis[(1E)-N-(anilinocarbothioyl)ethanehydrazonoyl]iron(II), bis[(E)-(aminocarbothioyl)hydrazono](phenyl)methyl]iron(II), iron(III) i-propoxide, iron(II) acetate, iron(III) oxo acetate perchlorate, ammonium iron(III) citrate, iron(II) acetylacetonate, iron(III) acetylacetonate, iron(II) bromide, iron(III) bromide, iron(II) chloride, iron(III) chloride, iron(II) arsenide, iron(III) arsenide, iron(III) citrate, iron(II) phthalocyanine bis(pyridine) complex, iron(II) ethylendiammoniumsulfate, iron(II) oxalate, iron(III) oxalate, ammonium iron(III) oxalate, iron(II) fluoride, iron(III) fluoride, iron(II) fumarate, iron(II) gluconate, iron(II) iodide, iron(III) iodide, iron(II) lactate, iron(III) nitrate, iron(II) phthalocyanine, iron(III) phthalocyanine-4, 4', 4", 4"'-tetrasulfonic acid, iron(III) phthalocyanine chloride, iron(II) perchlorate, iron(III) perchlorate, iron(II) sulphate, iron(III) sulphate, ammonium iron(II) sulphate, ammonium iron(III) sulphate, iron(II) phosphate, iron(III) phosphate, iron(III) tartrate, (+)-iron(II) ascorbate, iron(II) stearate, iron(II) sulfamate, iron(II) tetrafluoroborate, tetraethylammonium tetrachloroiron(III), tris(dibenzoylmethanato)iron(III), tris(ethylenediamine)iron(II) sulfate, iron(III) p-toluenesulfonate, iron(II) trifluoromethanesulfonate, iron(III) trifluoromethanesulfonate, iron(III) trifluoroacetylacetonate, bis(o-phenanthroline)iron(II) cyanide, bis(hexafluoroacetylacetonato)-(N,N,N',N'-tetramethylethylenediamine)iron (II), ethylenediaminetetraacetic acid, iron(III) sodium salt, diethylenetriaminepentaacetic acid iron(III) disodium salt, bis(N,N'-di-t-butyl acetamidina-to)iron(II), iron 2-ethylhexanoate, iron(III) naphthenate, tris(2,2,6,6-tetramethyl-3,5-heptanedionato)iron(III) and mixtures thereof.

5. The process according to any of claims 1 to 4, wherein the catalyst is present in an amount of 0.1 to 50 µmol iron per mol 2-mercaptoethanol.

6. The process according to any of claims 1 to 5, wherein the at least one tertiary amine contains three identical or different, unbranched or branched alkyl radicals having 1 to 20 carbon atoms in each case, where individual carbon atoms can also be, independently of another, replaced by a hetero atom selected from the group consisting of N or O and two or three radicals can also be joined to one another to form a chain comprising at least four atoms.

7. The process according to any of claims 1 to 6, wherein the at least one tertiary amine is selected from the group consisting of trimethyl amine, triethyl amine, tripropyl amine, triisopropyl amine, ethyl diisopropyl amine, tri-n-butyl amine, tripentyl amine, trihexyl amine, tricyclohexyl amine, triisoamyl amine, trioctyl amine, tris(2-ethylhexyl) amine, tristearyl amine, trioleyl amine, tridecyl amine, dimethyl stearyl amine, N,N-dimethyl benzyl amine, N,N-dibutyl benzyl amine, N,N-dimethyl aniline, N,N-dihexyl aniline, N,N-diethyl aniline, N,N-dimethyltoluidine, pyridine, quinoline, picoline, 2,4-lutidine, 2,6-lutidine, trimethylpyridine, 2-methyl-5-ethylpyridine (collidine), N-methylpiperidine, N,N'-dimethylpiperazine, N-methyl morpholine, N-methyl pyrrolidine, sparteine, tris(2-hydroxyethyl) amine, tris(2-hydroxypropyl) amine, methyl di(2-hydroxyethyl) amine, (N,N-Dimethylaminopropyl)-acetamide, octyldiethyl amine, N-octyl-N-hydroxyethylmethylamine, N,N-didecylmethyl amine, N-dodecyl-N-tetradecylhydroxyethylamine, N,N-ditetradecylmethylamine, N-tetradecyldimethylamine, N-hexadecyl-N-ethylmethylamine, N-octadecyl-N-eicosylmethylamine, N-docosyldimethylamine, N-tetracosyldimethylamine, triethylenediamine, tetramethyl guanidine, DABCO, *N,N,N,N',N'-*Pentamethyldiethylenetriamine, *N,N,N,N*-Tetraethyl-1,3-propanediamine, *N,N,N,N-*Tetramethyl-1,4-butanediamine, *N,N,N,N-*Tetramethyl-2-butene-1,4-diamine, *N,N,N,N-*Tetramethyl-1,6-hexanediamine, 1,4,8,11-Tetramethyl-1,4,8,11-tetraazacyclotetradecane, 1,3,5-Trimethylhexahydro-1,3,5-triazine, DBU, DBN and mixtures thereof.

8. The process according to any of claims 1 to 7, wherein the reaction mixtures comprises 2-mercaptoethanol, bis-(2-hydroxyethyl)-disulfide, at least one tertiary amine, at least one iron comprising salt or complex and oxygen.

9. The process according to claim 8, wherein the reaction mixture consists of 2-mercaptoethanol, bis-(2-hydroxyethyl)-disulfide, at least one tertiary amine, at least one iron comprising salt or complex, water, oxygen and optionally further components.

10. The process according to any of claims 1 to 9, wherein it is conducted at a temperature of 0 to 100 °C.

11. The process according to any of claims 1 to 10, wherein it is conducted at a partial pressure of oxygen of 0.2 to 20 bar (a).

## Patentansprüche

1. Verfahren zur Herstellung von Bis(2-hydroxyethyl)disulfid durch Oxidation von 2-Mercaptoethanol mit Sauerstoff in einer Reaktionsmischung, die mindestens ein homogen verteiltes eisenhaltiges Salz oder mindestens einen homogen verteilten eisenhaltigen Komplex als Katalysator und mindestens ein tertiäres Amin umfasst.

2. Verfahren nach Anspruch 1, bei dem das mindestens eine Eisensalz bzw. der mindestens eine Eisenkomplex aus organischen oder anorganischen Eisensalzen oder -komplexen ausgewählt wird.

3. Verfahren nach Anspruch 1 oder 2, bei dem das mindestens eine Eisensalz Eisen in der Oxidationsstufe +2 und/oder +3 umfasst.

4. Verfahren nach einem der Ansprüche 1 bis 3, bei dem das mindestens eine Eisensalz bzw. der mindestens eine Eisenkomplex, das bzw. der gegebenenfalls mindestens einen neutralen Liganden, wie Wasser, enthält, aus der Gruppe bestehend aus Eisen(II)-oxid, Eisen(III)-oxid, Eisen(II,III)-oxid, Eisen(II)-sulfid, Eisen(II)-disulfid, Eisen(II,III)-sulfid, Lithiumeisen(II)-phosphat, Lithiumeisen(III)-oxid, Eisen(II)-phosphid, Eisen(III)-phosphid, Eisen(III)-pyrophosphat, Eisen(III)-phosphat, Eisen(III)-Ionophor IV, Eisen(II)-molybdat, Ammonium-eisen(III)-hexacyanoferrat(II), Eisen(III)-ferrocyanid, 5,10,15,20-Tetrakis(pentafluorphenyl)-21H,23H-porphyrineisen(III)-chlorid, 5,10,15,20-Tetraphenyl-21H,23H-porphineisen(III)-chlorid, 5,10,15,20-Tetrakis(4-methoxyphenyl)-21H,23H-porphineisen(III)-chlorid, 2,3,7,8,12,13,17,18-Octaethyl-21H,23H-porphineisen(III)-acetat, 2,3,7,8,12,13,17,18-Octaethyl-21H,23H-porphineisen(III)-chlorid, Acetylcyclopentadienyleisen(II)-carbonyltriphenylphosphin-Komplex, [N,N'-Bis(2-pyridylmethyl)]-2,2'-bipyrrolidinbis(acetonitril)eisen(II)-hexafluoroantimonat, Eisen(II)-titanat, Eisen(II)-silicid, Eisen(III)-Ionophor VI, Bis(cyclopentadienyl)eisen(II), Cyclopentadienyl(formylcyclopentadienyl)eisen(II), (Boronocyclopentadienyl)-cyclopentadienyleisen(II), Bis(cyclopentadienyl)eisen(III)-tetrafluoroborat, Cyclopentadienyl[(hydroxymethyl)cyclopentadienyl]-eisen(II), Bis(acetylcyclopentadienyl)eisen(II), Bis(methylcyclo-pentadienyl)eisen(II), Bis(ethylcyclopentadienyl)eisen(II), Bis(i-propylcyclopentadienyl)eisen(II), Dichlorobis[o-phenylenbis(diphenyl-phosphin)]eisen(II), Cyclopentadienyleisen(II)-dicarbonyliodid, Bis(pentamethylcyclopentadienyl)eisen(II), Bis(tetramethylcyclopenta-dienyl)eisen(II), Cyclopentadienyldicarbonyl(tetrahydrofuran)eisen(II)-tetrafluoroborat, Tricarbonyl(cyclooctatetraen)eisen(II), Cyclopentadienyleisen(II)-dicarbonyl-Dimer, Cyclopentadienyl-(fluoren)eisen(II)-hexafluorophosphat, (Nicotinamido-methyl)phosphonsäure-Eisen(II)-Salz, Tricarbonyl(2-methoxycyclohexadienylium)eisen(II)-hexafluorophosphat, Tricarbonyl(4-methoxy-1-methylcyclohexadienylium)eisen(II)-tetrafluoroborat, meso-Tetra(4-N-methypyridyl)porphyrineisen(III), 4-Dimethylaminopyridinyl(pentaphenylcyclopentadienyl)eisen(II), 4-Pyrrolidinopyridinyl(pentamethylcyclopentadienyl)eisen(II), 2,6-Bis-[1-(2,6-diisopropylphenylimino)ethyl]pyridineisen(II)-chlorid, (Aminomethyl)phosphonsäure-Eisen(II)-Salz, Bis[(1E)-N-(aminocarbothioyl)ethanhydrazonoyl]eisen(II), Bis[(1E)-N-(anilinocarbothioyl)ethanhydrazonoyl]eisen(II), Bis[(E)-(amino-carbothioyl)hydrazono](phenyl)methyl]eisen(II), Eisen(III)-i-propoxid, Eisen(II)-acetat, Eisen(III)-oxoacetatperchlorat, Ammoniumeisen(III)-citrat, Eisen(II)-acetylacetonat, Eisen(III)-acetylacetonat, Eisen(II)-bromid, Eisen(III)-bromid, Eisen(II)-chlorid, Eisen(III)-chlorid, Eisen(II)-arsenid, Eisen(III)-arsenid, Eisen(III)-citrat, Eisen(II)-phthalocyaninbis(pyridin)-Komplex, Eisen(II)-ethylendiammonium-sulfat, Eisen(II)-oxalat, Eisen(III)-oxalat, Ammoniumeisen(III)-oxalat, Eisen(II)-fluorid, Eisen(III)-fluorid, Eisen(II)-fumarat, Eisen(II)-gluconat, Eisen(II)-iodid, Eisen(III)-iodid, Eisen(II)-lactat, Eisen(III)-nitrat, Eisen(II)-phthalocyanin, Eisen(III)-phthalocyanin-4,4',4",4'"-tetrasulfonsäure, Eisen(III)-phthalocyaninchlorid, Eisen(II)-perchlorat, Eisen(III)-perchlorat, Eisen(II)-sulfat, Eisen(III)-sulfat, Ammoniumeisen(II)-sulfat, Ammoniumeisen(III)-sulfat, Eisen(II)-phosphat, Eisen(III)-phosphat, Eisen(III)-tartrat, (+)-Eisen(II)-ascorbat, Eisen(II)-stearat, Eisen(II)-sulfamat, Eisen(II)-tetrafluoroborat, Tetraethylammoniumtetrachloroeisen(III), Tris-(dibenzoylmethanato)eisen(III), Tris(ethylendiamin)eisen(II)-sulfat, Eisen(III)-*p*-toluolsulfonat, Eisen(II)-trifluormethansulfonat, Eisen-(III)-trifluormethansulfonat, Eisen(III)-trifluoracetylacetonat, Bis(o-phenanthrolin)eisen(II)-cyanid, Bis(hexafluoracetylacetonato)-(N,N,N',N'-tetramethylethylendiamin)eisen(II), Ethylendiamintetraessigsäure-Eisen(III)-Natrium-Salz, Diethylentriaminpenta-essigsäureeisen(III)-Dinatriumsalz, Bis(N,N'-di-t-butylacetamidinato)-eisen(II), Eisen-2-ethylhexanoat, Eisen(III)-naphthenat, Tris(2,2,6,6-tetramethyl-3,5-heptandionato)eisen(III) und Mischungen davon ausgewählt wird.

5. Verfahren nach einem der Ansprüche 1 bis 4, bei dem der Katalysator in einer Menge von 0,1 bis 50 µmol Eisen pro Mol 2-Mercaptoethanol vorliegt.

6. Verfahren nach einem der Ansprüche 1 bis 5, bei dem das mindestens eine tertiäre Amin drei gleiche oder verschiedene, unverzweigte oder verzweigte Alkylreste mit jeweils 1 bis 20 Kohlenstoffatomen enthält, wobei einzelne Kohlenstoffatome auch unabhängig voneinander durch ein Heteroatom aus der Gruppe bestehend aus N oder O ersetzt sein können und zwei oder drei Reste auch miteinander zu einer Kette mit mindestens vier Atomen verbunden sein können.

7. Verfahren nach einem der Ansprüche 1 bis 6, bei dem das mindestens eine tertiäre Amin aus der Gruppe bestehend aus Trimethylamin, Triethylamin, Tripropylamin, Triisopropylamin, Ethyldiisopropylamin, Tri-n-butylamin, Tripentylamin, Trihexylamin, Tricyclohexylamin, Triisoamylamin, Trioctylamin, Tris(2-ethylhexyl)amin, Tristearylamin, Trioleylamin, Tridecylamin, Dimethylstearylamin, N,N-Dimethylbenzylamin, N,N-Dibutylbenzylamin, N,N-Dimethylanilin, N,N-Dihexylanilin, N,N-Diethylanilin, N,N-Dimethyltoluidin, Pyridin, Chinolin, Picolin, 2,4-Lutidin, 2,6-Lutidin, Trimethylpyridin, 2-Methyl-5-ethylpyridin (Collidin), N-Methylpiperidin, N,N'-Dimethylpiperazin, N-Methylmorpholin, N-Methylpyrrolidin, Spartein, Tris(2-hydroxyethyl)amin, Tris(2-hydroxypropyl)amin, Methyldi(2-hydroxyethyl)amin, (N,N-Dimethylaminopropyl)acetamid, Octyldiethylamin, N-Octyl-N-hydroxyethylmethylamin, N,N-Didecylmethylamin, N-Dodecyl-N-tetradecylhydroxyethylamin, N,N-Ditetradecylmethylamin, N-Tetradecyldimethylamin, N-Hexadecyl-N-ethylmethylamin, N-Octadecyl-N-eicosylmethylamin, N-Docosyldimethylamin, N-Tetracosyldimethylamin, Triethylendiamin, Tetramethylguanidin, DABCO, *N,N,N,N',N'*-Pentamethyl-diethylentriamin, *N,N,N,N-*Tetraethyl-1,3-propandiamin, *N,N,N,N-*Tetramethyl-1,4-butandiamin, *N,N,N,N-*Tetramethyl-2-buten-1,4-diamin, *N,N,N,N-*Tetramethyl-1,6-hexandiamin, 1,4,8,11-Tetramethyl-1,4,8,11-tetraazacyclotetradecan, 1,3,5-Trimethylhexahydro-1,3,5-triazin, DBU, DBN und Mischungen davon ausgewählt wird.

8. Verfahren nach einem der Ansprüche 1 bis 7, bei dem die Reaktionsmischung 2-Mercaptoethanol, Bis(2-hydroxyethyl)disulfid, mindestens ein tertiäres Amin, mindestens ein eisenhaltiges Salz oder mindestens einen eisenhaltigen Komplex und Sauerstoff umfasst.

9. Verfahren nach Anspruch 8, bei dem die Reaktionsmischung aus 2-Mercaptoethanol, Bis(2-hydroxyethyl)disulfid, mindestens einem tertiären Amin, mindestens einem eisenhaltigen Salz oder Komplex, Wasser, Sauerstoff und gegebenenfalls weiteren Komponenten besteht.

10. Verfahren nach einem der Ansprüche 1 bis 9, bei dem es bei einer Temperatur von 0 bis 100 °C durchgeführt wird.

11. Verfahren nach einem der Ansprüche 1 bis 10, bei dem es bei einem Sauerstoff-Partialdruck von 0,2 bis 20 bar (a) durchgeführt wird.

## Revendications

1. Procédé de préparation de disulfure de bis-(2-hydroxyéthyle) par oxydation de 2-mercaptoéthanol avec de l'oxygène dans un mélange de réaction comprenant au moins un sel ou complexe comprenant du fer distribué de façon homogène en tant que catalyseur et au moins une amine tertiaire.

2. Procédé selon la revendication 1, dans lequel l'au moins un sel ou complexe de fer est choisi parmi des sels ou complexes de fer organiques ou inorganiques.

3. Procédé selon la revendication 1 ou 2, dans lequel l'au moins un sel de fer comprend du fer à l'état d'oxydation +2 et/ou +3.

4. Procédé selon l'une quelconque des revendications 1 à 3, dans lequel l'au moins un sel ou complexe de fer, contenant facultativement au moins un ligand neutre, tel que l'eau, est choisi dans le groupe constitué des oxyde de fer(II), oxyde de fer(III), oxyde de fer(II,III), sulfure de fer(II), disulfure de fer(II), sulfure de fer(II,III), phosphate de lithium-fer(II), oxyde de lithium-fer(III), phosphure de fer(II), phosphure de fer(III), pyrophosphate de fer(III), phosphate de fer(III), ionophore IV-fer(III), molybdate de fer(II), hexacyanoferrate(II) d'ammonium-fer(III), ferrocyanure de fer(III), chlorure de 5,10,15,20-tétrakis-(pentafluorophényl)-21H,23H-porphyrine-fer(III), chlorure de 5,10,15,20-tétraphényl-21H,23H-porphine-fer(III), chlorure de 5,10,15,20-tétrakis(4-méthoxyphényl)-21H,23H-porphine-fer(III), acétate de 2,3,7,8,12,13,17,18-octaéthyl-21h,23h-porphine-fer(III), chlorure de 2,3,7,8,12,13,17,18-octaéthyl-21H,23H-porphine-fer(III), complexe acétyl-cyclopenta-diényl-fer(II)-carbonyl-triphénylphosphine, hexafluoroantimonate de [N,N'-bis(2-pyridylméthyl)]-2,2'-bipyrrolidinebis(acétonitrile)fer(II), titanate de fer(II), siliciure de fer(II), ionophore VI-fer(III), bis(cyclopentadiényl)-fer(II), cyclopentadiényl-(formylcyclopentadiényl)fer(II), (borocyclopenta-diényl)cyclopentadiényl-fer(II), tétrafluoroborate de bis(cyclopentadiényl)fer(III), cyclopentadiényl-[(hydroxyméthyl)cyclopentadiényl]fer(II), bis(acétyl-cyclopentadiényl)fer(II), bis(méthylcyclopentadiényl) fer(II), bis(éthylcyclopentadiényl)fer(II), bis(i-propylcyclopentadiényl)fer(II), dichloro-bis[o-phénylènebis(diphénylphosphine)]fer(II), iodure de cyclopentadiényl-fer(II)-dicarbonyle, bis(pentaméthyl-cyclopentadiényl)fer(II), bis(tétraméthylcyclopenta-diényl)fer(II), cyclopentadiényldicarbonyl-(tétrahydrofurane)fer(II), tétrafluoroborate de tricarbonyl(cyclooctatétraène)fer(II), dimère de cyclopentadiényle fer(II)-dicarbonyle, hexafluorophosphate de cyclopentadiényl(fluorène)fer(II), sel de fer(II) d'acide (nicotinamidométhyl)phosphonique, hexafluorophosphate de tricarbonyl(2-méthoxycyclohexadiénylium)-fer(II), tétrafluoroborate de tricarbonyl(4-méthoxy-1-méthylcyclohexadiénylium)-fer(II), méso-tétra(4-N-méthypyridyl)porphyrine-fer(III), 4-diméthylaminopyridinyl(pentaphénylcyclo-pentadiényl)fer(II), 4-pyrrolidinopyrindinyl-(pentaméthylcyclopentadiényl)fer(II), chlorure de 2,6-bis-[1-(2,6-diisopropylphénylimino)éthyl]pyridine-fer(II), sel de fer(II) d'acide (aminométhyl)-phosphonique, bis[(1E)-N-(aminocarbothioyl)éthane-hydrazonoyl]fer(II), bis[(1E)-N-(anilinocarbothioyl)-éthane-hydrazonoyl]fer(II), bis[(E)-(aminocarbothioyl)-hydrazono](phényl)méthyl]fer(II), i-propoxyde de fer(III), acétate de fer(II), oxoacétate-perchlorate de fer(III), citrate d'ammonium-fer(III), acétylacétonate de fer(II), acétylacétonate de fer(III), bromure de fer(II), bromure de fer(III), chlorure de fer(II), chlorure de fer(III), arséniure de fer(II), arséniure de fer(III), citrate de fer(III), complexe fer(II)-phtalocyanine-bis(pyridine), sulfate d'éthylène-diammonium-fer(II), oxalate de fer(II), oxalate de fer(III), oxalate d'ammonium-fer(III), fluorure de fer(II), fluorure de fer(III), fumarate de fer(II), gluconate de fer(II), iodure de fer(II), iodure de fer(III), lactate de fer(II), nitrate de fer(III), phtalocyanine-fer(II), acide phtalocyanine-4,4',4",4"'-tétrasulfonique-fer(III), chlorure de fer(III)-phtalocyanine, perchlorate de fer(II), perchlorate de fer(III), sulfate de fer(II), sulfate de fer(III), sulfate d'ammonium-fer(II), sulfate d'ammonium-fer(III), phosphate de fer(II), phosphate de fer(III), tartrate de fer(III), (+)-ascorbate de fer(II), stéarate de fer(II), sulfamate de fer(II), tétrafluoroborate de fer(II), tétraéthylammonium-tétrachloro-fer(III), tris(dibenzoylméthanato)fer(III), sulfate de tris(éthylènediamine)fer(II), p-toluènesulfonate de fer(III), trifluorométhanesulfonate de fer(II), trifluorométhanesulfonate de fer(III), trifluoroacétylacétonate de fer(III), cyanure de bis(o-phénanthroline)fer(II), bis(hexafluoroacétylacétonato)-(N,N,N',N'-tétraméthyléthylènediamine)fer(II), sel de sodium d'acide éthylènediaminetétraacétique-fer(III), sel disodique d'acide diéthylènetriaminepentaacétique-fer(III), bis(N,N'-di-t-butylacétamidinato)fer(II), 2-éthylhexanoate de fer, naphténate de fer(III), tris(2,2,6,6-tétraméthyl-3,5-heptanedionato)fer(III) et des mélanges de ceux-ci.

5. Procédé selon l'une quelconque des revendications 1 à 4, dans lequel le catalyseur est présent en une quantité de 0,1 à 50 µmol de fer par mole de 2-mercaptoéthanol.

6. Procédé selon l'une quelconque des revendications 1 à 5, dans lequel l'au moins une amine tertiaire contient trois radicaux alkyle non ramifiés ou ramifiés identiques ou différents ayant 1 à 20 atomes de carbone dans chaque cas, où les atomes de carbone individuels peuvent également être, indépendamment les uns des autres, remplacés par un hétéroatome choisi dans le groupe constitué de N ou O et deux ou trois radicaux peuvent également être assemblés mutuellement pour former une chaîne comprenant au moins quatre atomes.

7. Procédé selon l'une quelconque des revendications 1 à 6, dans lequel l'au moins une amine tertiaire est choisie dans le groupe constitué des triméthylamine, triéthylamine, tripropylamine, triisopropylamine, éthyldiisopropylamine, tri-n-butylamine, tripentylamine, trihexylamine, tricyclohexylamine, triisoamylamine, trioctylamine, tris(2-éthylhexyl)amine, tristéarylamine, trioléylamine, tridécylamine, diméthylstéarylamine, N,N-diméthylbenzylamine, N,N-dibutylbenzylamine, N,N-diméthylaniline, N,N-dihexylaniline, N,N-diéthylaniline, N,N-diméthyltoluidine, pyridine, quinoléine, picoline, 2,4-lutidine, 2,6-lutidine, triméthylpyridine, 2-méthyl-5-éthylpyridine (collidine), N-méthylpipéridine, N,N'-diméthylpipérazine, N-méthylmorpholine, N-méthyl-pyrrolidine, spartéine, tris(2-hydroxyéthyl)amine, tris(2-hydroxypropyl)amine, méthyl-di(2-hydroxyéthyl)-amine, (N,N-diméthylaminopropyl)acétamide, octyl-diéthylamine, N-octyl-N-hydroxyéthylméthylamine, N,N-didécylméthylamine, N-dodécyl-N-tétradécylhydroxy-éthylamine, N,N-ditétradécylméthylamine, N-tétra-décyldiméthylamine, N-hexadécyl-N-éthylméthylamine, N-octadécyl-N-eicosylméthylamine, N-docosyldiméthyl-amine, N-tétracosyldiméthylamine, triéthylènediamine, tétraméthylguanidine, DABCO, *N,N,N,N',N'*-pentaméthyl-diéthylènetriamine, *N,N,N,N*-tétraéthyl-1,3-propane-diamine, *N,N,N,N*-tétraméthyl-1,4-butanediamine, *N,N,N,N-*tétraméthyl-2-butène-1,4-diamine, *N,N,N,N*-tétraméthyl-1,6-hexanediamine, 1,4,8,11-tétraméthyl-1,4,8,11-tétra-azacyclotétradécane, 1,3,5-triméthylhexahydro-1,3,5-triazine, DBU, DBN et des mélanges de ceux-ci.

8. Procédé selon l'une quelconque des revendications 1 à 7, dans lequel le mélange de réaction comprend du 2-mercaptoéthanol, du disulfure de bis-(2-hydroxyéthyle), au moins une amine tertiaire, au moins un sel ou complexe comprenant du fer et de l'oxygène.

9. Procédé selon la revendication 8, dans lequel le mélange de réaction est constitué de 2-mercaptoéthanol, disulfure de bis-(2-hydroxyéthyle), au moins une amine tertiaire, au moins un sel ou complexe comprenant du fer, de l'eau, de l'oxygène et facultativement d'autres composants.

10. Procédé selon l'une quelconque des revendications 1 à 9, où celui-ci est conduit à une température de 0 à 100 °C.

11. Procédé selon l'une quelconque des revendications 1 à 10, où celui-ci est conduit à une pression partielle d'oxygène de 0,2 à 20 bar (a).
